(19) Europäisches Patentamt
European Patent Office
Office européen des brevets

(11) Veröffentlichungsnummer: **0 000 392**
**B1**

(12) **EUROPÄISCHE PATENTSCHRIFT**

(45) Veröffentlichungstag der Patentschrift: **28.04.82**

(21) Anmeldenummer: **78100352.0**

(22) Anmeldetag: **11.07.78**

(51) Int. Cl.³: **C 07 D 501/20**, C 07 D 499/70, A 23 K 1/17, A 61 K 31/545, A 61 K 31/43

# (54) Cephalosporin und Penicillin-derivate, Verfahren zu deren Herstellung, und deren pharmazeutische Präparate.

(30) Priorität: **16.07.77 DE 2732283**

(43) Veröffentlichungstag der Anmeldung:
**24.01.79 Patentblatt 79/2**

(45) Bekanntmachung des Hinweises auf die Patenterteilung:
**28.04.82 Patentblatt 82/17**

(84) Benannte Vertragsstaaten:
**BE CH DE FR GB NL SE**

(56) Entgegenhaltungen:
**FR - A - 2 292 471**
**FR - A - 2 359 145**

(73) Patentinhaber: **BAYER AG**
**Zentralbereich Patente, Marken und Lizenzen**
**D-5090 Leverkusen 1, Bayerwerk (DE)**

(72) Erfinder: **Preiss, Michael, Dr**
**Egenstrasse 21**
**D-5600 Wuppertal 1 (DE)**
Erfinder: **König, Hans-Bodo, Dr.**
**Herberts Katernberg 10**
**D-5600 Wuppertal 1 (DE)**
Erfinder: **Metzger, Karl Georg, Dr.**
**Pahlkestrasse 15**
**D-5600 Wuppertal 1 (DE)**
Erfinder: **Feyen, Peter, Dr.**
**Mozartstrasse 1**
**D-4020 Mettmann (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 392 B1

Cephalosporin- und Penicillin-derivate, Verfahren zu deren Herstellung, und deren pharmazeutische Präparate

Die vorliegende Erfindung betrifft neue β-Lactam-Verbindungen, Verfahren zu ihrer Herstellung sowie ihre Verwendung als Arzneimittel, insbesondere als antimikrobielle Mittel und als Mittel zur Förderung des Wachstums und zur Verbesserung der Futterverwendung bei Tieren.

Es ist bereits bekannt geworden, daß bestimmte α-(Imidazolidin-2-oxo-1-yl-carbonylamino)-benzylpenicilline antibakteriell wirksam sind (vgl. BE—PS 767 647 und 767 648, DT—OS 2 152 968 und FR—PS 2 292 471).

Die erfindungsgemäßen neuen β-Lactam-Antibiotica unterscheiden sich chemisch von den bekannten Verbindungen des Standes der Technik vor allem durch den α-ständigen heterocyclischen Ring in der Acylseitenkette, wodurch sie überraschenderweise eine verbesserte Wirksamkeit zeigen.

Die Erfindung betrifft neue β-Lactam-Verbindungen der Formel (I)

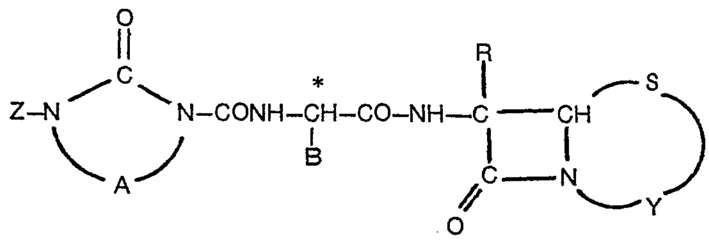

(I)

in welcher

Z 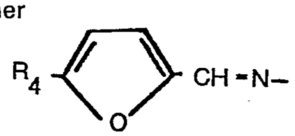 mit $R_4 = H, CH_3$, Cl und Br

R für Wasserstoff oder Niederalkoxy steht,

A für $—CH_2—CH_2—$, $—CH_2—CH_2—CH_2—$ oder 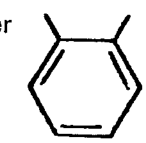 steht,

B für einen Furyl-, Thienyl-, 1,3,5-Trimethyl-pyrazolyl- oder 2-Aminothiazolylrest steht, und

Y für die Gruppen 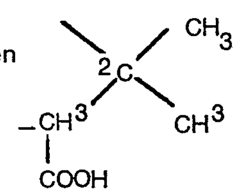 und 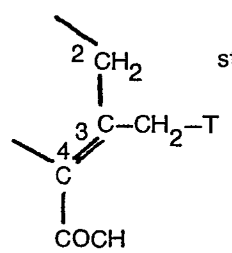 steht,

in welchem das Kohlenstoffatom, welches die Gruppe —COOH trägt, an das Stickstoffatom des β-Lactam-ringes gebunden ist und

T für Wasserstoff, $—O—CH—CH_3$, Hydroxyl oder für gegebenenfalls durch Alkyl mit 1 bis 4 Kohlenstoffatomen oder $CF_3$ substituiertes Thiadiazolylthio oder Tetrazolylthio steht, wobei diese Verbindungen der Formel I bezüglich des Chiralitätszentrums $\overset{*}{C}$ in den beiden möglichen R- und S-Konfigurationen sowie als Gemische der daraus resultierenden Diastereomeren vorliegen können, und wobei die Verbindungen der Formel I bezüglich der Iminogruppe im Rest Z sowohl in der syn-Form als auch in der anti-Form vorliegen können und wobei diese Verbindungen der Formel I auch in den verschiedenen Hydratformen sowie in Form ihrer pharmazeutisch verwendbaren Salze vorliegen können.

Weiterhin wurde gefunden, daß man die neuen β-Lactam-Antibiotica der Formel I erhält, wenn man Verbindungen der Formel II

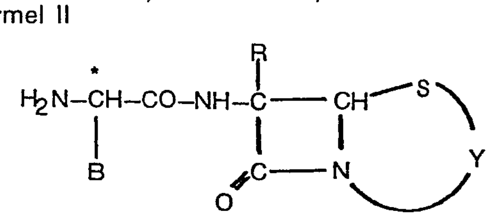

(II)

in welcher

R, B, C*, X und Y die oben angegebene Bedeutung haben oder deren Salze, mit Verbindungen der Formel III

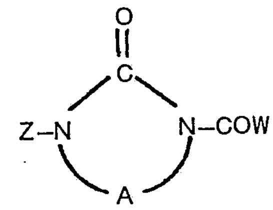

(III)

in welcher

Z und A die oben angegebene Bedeutung haben und

W für Halogen, Azid oder eine andere nukleofuge Abgangsgruppe steht,

in Gegenwart eines Lösungsmittels und gegebenenfalls eines Säurebindemittels bei Temperaturen von etwa −20°C bis etwa +50°C umsetzt und die erhaltenen β-Lactam-Antibiotica gegebenenfalls in ihre pharmazeutisch verwendbaren Salze oder Ester überführt oder aus den erhaltenen Salzen gewünschtenfalls die freien Säuren hergestellt.

Die erfindungsgemäßen Verbindungen der Formel (I), in welcher R für Niederalkoxy steht, sind ebenfalls herstellbar durch Alkoxylierung der entsprechenden Wasserstoffderivate (R=H), wobei es vorteilhaft ist, bei der Alkoxylierung solche Verbindungen zu verwenden, in denen E eine geeignete Schutzgruppe bedeutet.

Die erfindungsgemäßen Verbindungen zeigen neben guter Verträglichkeit und Löslichkeit eine breite antibakterielle Wirkung, d.h. Wirkung gegenüber mehreren Bakterienfamilien im gram-negativen Bereich und gegenüber β-Lactamasebildnern. Aufgrund ihrer starken antibakteriellen Eigenschaften und aufgrund ihrer Fähigkeit, das Wachstum und die Futterverwertung bei Tieren zu verbessern, stellen die erfindungsgemäßen Verbindungen somit eine Bereicherung der Technik dar.

Verwendet man beispielsweise α-Amino-furfurylacetylpenicillin und 1-Chlor arbonyl-3-furfurylidenamino-imidazolidin-2-on als Ausgangsstoffe, so kann der Reaktionsablauf durch das folgende Formelschema wiedergegeben werden:

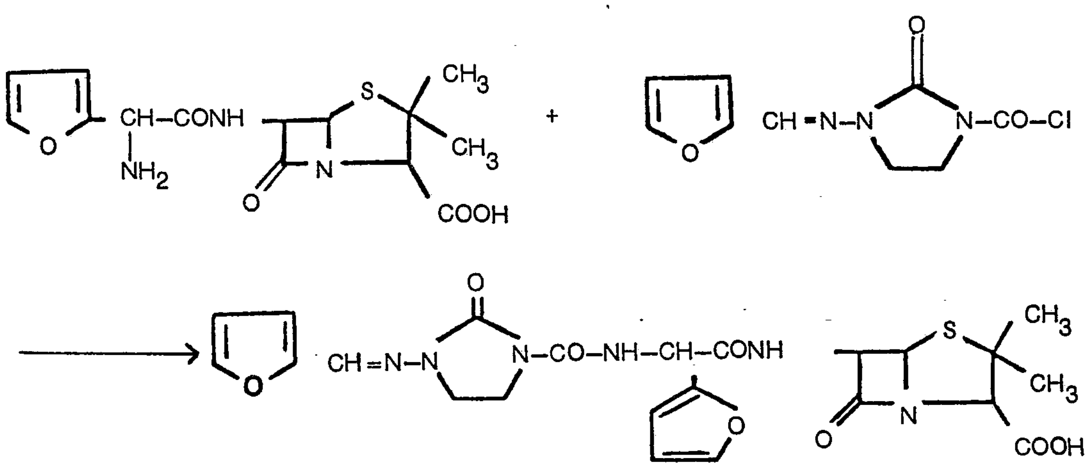

Bei den folgenden Darlegungen bedeutet der Ausdruck "Nieder-alkyl" überall, auch in Verbindung mit anderen Atomen oder Gruppen (z.B. Niederalkoxy, HCON-(Niederalkyl), usw.) geradkettiges oder verzweigtes Alkyl mit vorzugsweise 1 bis 6, insbesondere 1 bis 4 Kohlenstoffatomen. Beispielhaft seien gegebenenfalls substituiertes Methyl, Äthyl, n- und i-Propyl, n-, i- und t-Butyl genannt. "Niederalkyl" kann durch 1 bis 5, insbesondere 1 bis 3 gleiche oder verschiedene Halogenatome, wobei als Halogenatome vorzugsweise Fluor, Chlor und Brom, insbesondere Fluor und Chlor stehen, substituiert sein. Beispielhaft seien Trifluormethyl, Chlor-di-fluormethyl, Brom-methyl, 2,2,2-Tri-fluoräthyl und Pentafluoräthyl genannt.

In der Definition von T bedeutet Alkyl in Alkyl-CO—O— vorzugsweise Alkyl mit 1 bis 4, insbesondere 1 oder 2 Kohlenstoffatome. Beispielhaft seien Methyl und Äthyl genannt, wobei Methyl besonders bevorzugt ist.

Der heterocyclische Ring Het in —S—Het (Definition von T) besteht aus 5 oder 6 Ringgliedern und enthält 1 bis 4, vorzugsweise 1 bis 3 gleiche oder verschiedene Heteroatome, wobei als Heteroatome Sauerstoff, Schwefel und Stickstoff stehen. Bevorzugt ist der heterocyclische Ring ungesättigt und enthält besonders bevorzugt 2 Doppelbindungen. Der heterocyclische Ring kann einen oder mehrere, vorzugsweise 1 oder 2, insbesondere einen Substituenten enthalten. Als Substituenten seien beispielhaft aufgeführt: Halogen, wie Fluor, Chlor und Brom, vorzugsweise Chlor und Brom, Amino,

Niederalkylamino, Diniederalkylamino, Niederalkyl, Cycloalkyl (mit 3 bis 7, vorzugsweise 5 oder 6 Kohlenstoffatomen im Cycloalkylteil), Niederalkyloxy (Bedeutung von "Niederalkyl" siehe oben), Trifluormethyl, Phenyl, Benzyl und Acylamino mit vorzugsweise 2 bis 5, insbesondere 2 oder 3 Kohlenstoffatomen. Als —S—Het seien als besonders bevorzugt aufgeführt:

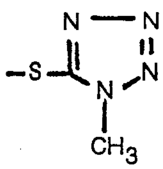 , 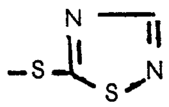 , 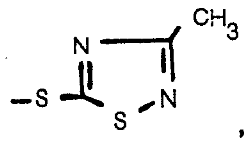 , 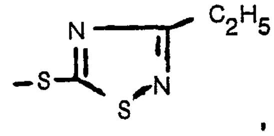 ,

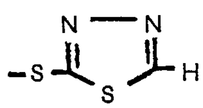 , 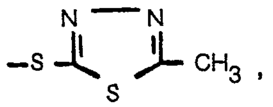 , 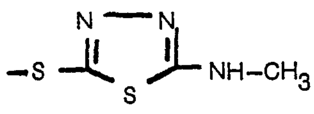 , 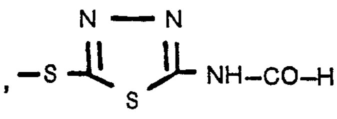 ,

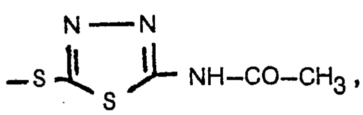 , 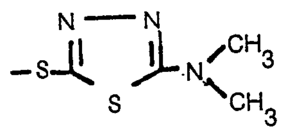 , 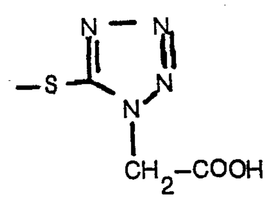

Der —S-Phenylrest in der Definition von T kann einen oder mehrere, vorzugsweise 1 bis 3, insbesondere 1 oder 2 gleiche oder verschiedene Substituenten tragen, wobei als Substituenten diejenigen bevorzugt werden, welche oben als mögliche Substituenten des Restes —S-Het aufgeführt werden.

R in der Bedeutung von Niederalkoxy bezeichnet bevorzugt eine Alkoxygruppe mit 1 bis 6, insbesondere 1 bis 4 Kohlenstoffatomen, insbesondere aber Methoxy oder Äthoxy.

Ganz besonders bevorzugt sind erfindungsgemäße Verbindungen, in welchen $\overset{*}{C}$ in der D— = R-Konfiguration vorliegt.

Alle Kristallformen und Hydratformen der erfindungsgemäßen Verbindungen der allgemeinen Formel (I) und ihrer Salze sind in gleicher Weise antibakteriell wirksam.

Halogen W steht für Fluor, Chlor und Brom, vorzugsweise für Brom oder Chlor, insbesondere für Chlor.

Unter nukleofugen Abgangsgruppen in der Definition von W sind alle üblicherweise in der organischen Chemie verwendeten nukleofugen Gruppen und vor allem solche zu verstehen, welche in Angewandte Chemie, *81* (1969), Seite 543 beschrieben sind.

Die Verbindungen der Formel (I) fallen bei der Herstellung vielfach in Form von Salzen an oder können leicht in diese überführt werden. Besonders wichtig für den Gebrauch als Arzneimittel sind die pharmazeutisch verwendbaren Salze der Verbindungen gemäß Formel (I).

Pharmazeutisch verwendbare Salze der Verbindungen der Formel (I) sind Salze dieser Verbindungen mit anorganischen und organischen Basen an der sauren Carboxylgruppe bzw. den sauren Carboxyl- und Sulfonsäuregruppen. Als Basen können hierzu alle in der pharmazeutischen Chemie, insbesondere in der Chemie der Antibiotika, üblicherweise verwendeten Basen eingesetzt werden. Als anorganische Basen seien beispielhaft genannt: Alkali- und Erdalkalihydroxide, Alkali- und Erdalkalicarbonate und Alkalihydrogen-carbonate, wie Natrium- und Kaliumhydroxid, Calcium- und Magnesiumhydroxid, Natrium- und Kaliumcarbonat, Calciumcarbonat, Natrium- und Kaliumhydrogencarbonat; Aluminiumhydroxid und Ammoniumhydroxid. Als organische Amine können primäre, sekundäre und tertiäre aliphatische Amine sowie heterocyclische Amine eingesetzt werden. Beispielhaft seien genannt: Di- und Triniedrigalkylamine, z.B. Diäthylamin, Triäthylamin, Tri-$\beta$-hydroxyäthylamin, Procain, Dibenzylamin, N,N'-Dibenzyläthylendiamin, N-Benzyl-$\beta$-phenyl-äthylamin, N-Methyl- und N-Äthylmorpholin, 1-Ephenamin, Dehydroabietylamin, N,N'-Bis-dehydroabietyläthylendiamin, N-Niedrigalkylpiperidin. Auch sogenannte basische Aminosäuren wie Lysin oder Arginin können vorteilhaft als Basen Verwendung finden. Besonders bevorzugte Salze sind die Natriumsalze.

Ganz besonders bevorzugte Verbindungen der Formel 1 sind solche, in welchen

R für Wasserstoff oder Methoxy und

A für —$CH_2$—$CH_2$—

Y für die Gruppen 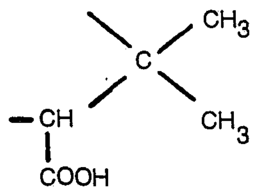 und 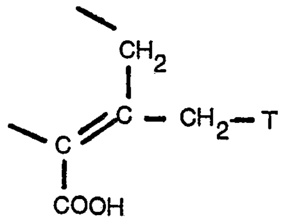 steht, wobei

T für Wasserstoff, $—O—CO—CH_3$, Hydroxyl oder für gegebenenfalls durch Alkyl mit 1 bis 4 Kohlenstoffatomen oder $CF_3$ substituiertes Thiadiazolylthio oder Tetrazolylthio steht; und

$\overset{*}{C}$ liegt in der D— = R-Konfiguration vor;

sowie die Natriumsalze dieser Verbindungen.

Die als Ausgangsstoffe verwendeten Verbindungen der allgemeinen Formel II sind bereits bekannt oder nach bekannten Methoden erhältlich.

Alle Kristallformen, Hydratformen und Salze der Verbindungen der allgemeinen Formel II sind als Ausgangsmaterialien für das erfindungsgemäße Verfahren geeignet.

Als Beispiele seien genannt:

$\alpha$-Amino-furfurylacetyl-penicillin, 7-($\alpha$-Amino-furfuryl-acetamido-3-acetoxymethyl-ceph-3-em-4-carbonsäure.

Als Salze der Verbindungen der Formel II können vorzugsweise Salze mit Basen eingesetzt werden, welche als für die Salzbildung mit Verbindungen der Formel I geeignet aufgeführt werden. Besonders bevorzugt sind die Natriumsalze.

Die als Ausgangstoffe verwendeten Verbindungen der allgemeinen Formel III sind nach bekannten Methoden erhältlich. Sie können z.B. auf folgendem Wege erhalten werden (vgl. auch J.A.C.S. 78 (1956) 5349):

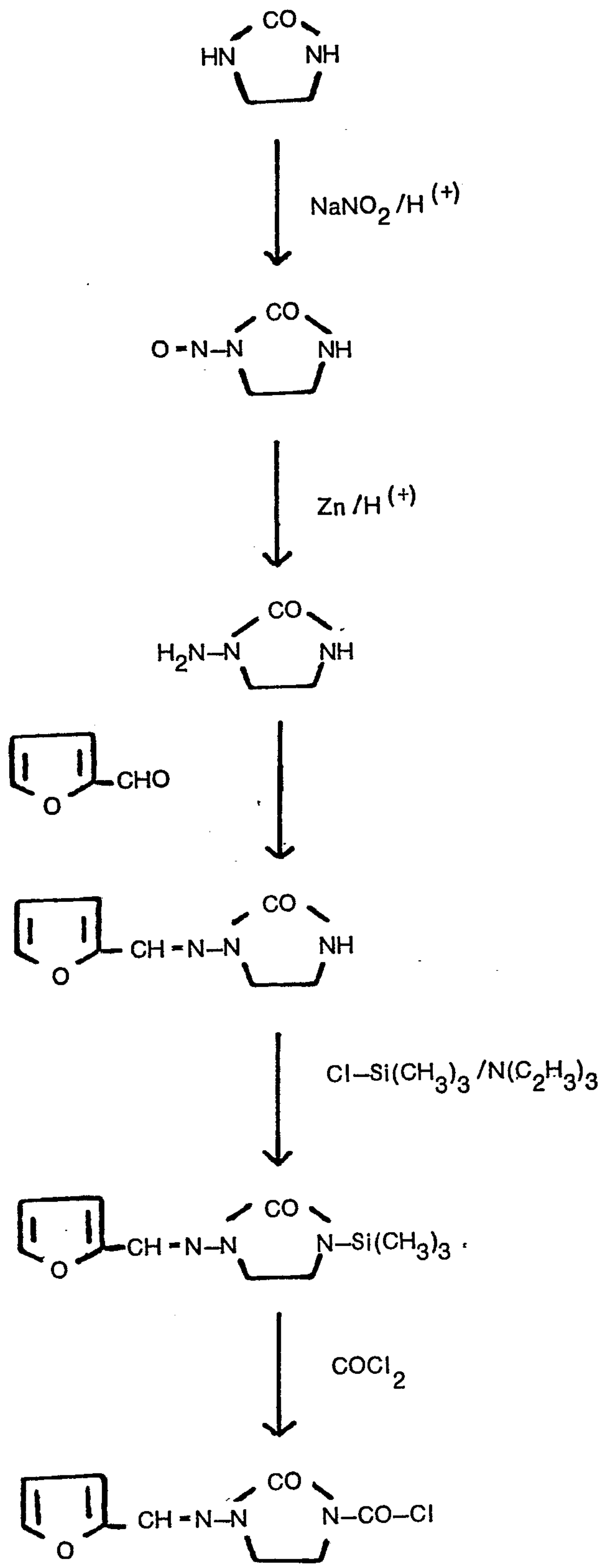

NaNO2/H(+)
Zn/H(+)
Cl–Si(CH3)3/N(C2H3)3
COCl2

Die Phosgenierung ist auch ohne vorherige Silylierung direkt in einem inerten organischen Lösungsmittel in Gegenwart einer Base möglich.

Als Beispiele für erfindungsgemäße Ausgangsverbindungen der allgemeinen Formel (II) seien genannt:

1-Chlorcarbonyl-2-oxo-3-furfurylidenamino-imidazolidin
1-Azidocarbonyl-2-oxo-3-furfurylidenamino-imidazolidin
1-Chlorcarbonyl-2-oxo-3-crotonylidenamino-imidazolidin

Diejenigen Verbindungen der allgemeinen Formel (III), in denen W Azid ist, werden in üblicher Weise z.B. aus den entsprechenden Verbindungen (III), in denen W Halogen ist, durch Umsetzung beispielsweise mit Alkaliaziden erhalten.

Die als Ausgangsstoffe eingesetzten Verbindungen der Formel II sind bekannt oder können nach an sich bekannten Methoden hergestellt werden (vgl. DOS 2 555 159).

Als Verdünnungsmittel kommen beim erfindungsgemäßen Verfahren Wasser sowie alle inerten organischen Lösungsmittel, vorzugsweise solche, welche mit Wasser mischbar sind, in Frage. Hierzu gehören vor allem niedere Dialkylketone, z.B. Aceton, Methylathylketon, cyclische Aether, z.B. Tetrahydrofuran und Dioxan; Nitrile, z.B. Acetonitril; niedere Dialkylformamide, z.B. Dimethylformamid; niedere Alkylalkohole, z.B. Aethanol und Isopropanol sowie Dimethylsulfoxid. Diese Losungsmittel können auch in Mischungen untereinander sowie in beliebigen Mischungen einzelner oder mehrerer dieser Lösungsmittel mit Wasser verwendet werden. Das erfindungsgemäße Verfahren kann also durchgeführt werden in Gegenwart von: (a) ausschließlich Wasser, (b) ausschließlich einem oder mehreren organischen Lösungsmitteln oder (c) Wasser und einem oder mehreren organischen Lösungsmitteln. Ist wegen des Vorhandenseins von Wasser eine pH-Messung wahrend der erfindungsgemaßen Reaktion möglich, wird der pH der Reaktionsmischung durch Zusatz von Basen oder durch Verwendung von Puffergemischen vorzugsweise zwischen 6,5 bis 7,5 gehalten. Das erfindungsgemäße Verfahren läßt sich aber auch sehr gut in einem anderen pH-Bereich, beispielsweise zwischen 4,5 und 9,0 oder bei pH 2,0 bis 4,5, durchführen. Ferner ist es möglich, die Reaktion in mit Wasser nicht mischbaren Lösungsmitteln, z.B. halogenierten Kohlenwasserstoffen, wie Chloroform oder Methylenchlorid, unter Zusatz von organischen Basen, vorzugsweise Niederalkylaminen, z.B. Triäthylamin, Diäthylamin oder cyclischen Basen, z.B. N-Äthylpiperidin durchzuführen. Weiterhin läßt sich die Reaktion in einer Mischung aus Wasser und einem mit Wasser nicht mischbaren Lösungsmittel, wie z.B. Niederalkyläthern, wie Diäthyläther, halogenierten Kohlenwasserstoffen, wie Chloroform und Methylenchlorid; Schwefelkohlenstoff; Isobutylmethylketon; Estern wie Essigsäureäthylester; aromatischen Kohlenwasserstoffen wie Benzol, ausführen, wobei es zweckmäßig ist, kräftig zu rühren und den pH-Wert durch Basenzusatz oder Verwendung von üblichen Pufferlösungen, z.B. Phosphat-, Acetat- oder Citratpuffer, zwischen 4,5 und 9,0 oder z.B. 2,0 und 4,5 zu halten. Man kann die Reaktion aber auch in Wasser allein in Abwesenheit von organischen Lösungsmitteln in Gegenwart einer organischen oder anorganischen Base oder unter Zusatz von üblichen Pufferstoffen durchführen.

Als Säurebindemittel können alle in der Chemie der Antibiotica üblicherweise verwendeten Säurebinder verwendet werden. Hierzu gehören anorganische Basen und organische Basen, welche z.B. durch sterische Hinderung schwer acylierbar sind. Als Beispiele für anorganische Basen seien Natrium- und Kaliumhydroxid genannt. Als organische Basen kommen praktisch alle nicht oder schwer acylierbaren offenkettigen oder cyclischen Amine und auch heteroaromatische Basen infrage. Als Basen seien beispielhaft tertiäre Amine, vorzugsweise Niederalkylamine, z.B. Triäthylamin und/oder cyclische Basen, z.B. Pyridin sowie als schwer acylierbares sekundäres Amin Dicyclohexylamin genannt.

Beim erfindungsgemäßen Verfahren ist der Zusatz einer Base nur dann erforderlich, wenn während der Reaktion saure Verbindungen entstehen, z.B. im Falle, daß W für Halogen oder Azid steht.

Die Reaktionstemperaturen können in einem größeren Bereich variiert werden. Im allgemeinen arbeitet man zwischen etwa —20°C und etwa +50°C, vorzugsweise zwischen 0 und +20°C. Wie bei den meisten chemischen Reaktionen können jedoch prinzipiell auch höhere oder niedrigere Temperaturen verwendet werden.

Die Umsetzung kann bei Normaldruck, aber auch bei vermindertem oder erhöhtem Druck ausgeführt werden. Im allgemeinen arbeitet man bei Normaldruck.

Bei der Durchführung der erfindungsgemäßen Verfahren können die Anteile der Reaktionspartner der Formel (II) und (III) in weiten Grenzen variiert werden, ohne daß das Ergebnis nachteilig beeinflußt wird. Die Ausgangsstoffe können z.B. in äquimolekularen Mengen miteinander zur Reaktion gebracht werden. Es kann jedoch zweckmäßig sein, einen der beiden Reaktionspartner im Überschuß zu verwenden, um sie die Reinigung oder Reindarstellung des gewünschter Penicillins zu erleichtern und die Ausbeute zu erhöhen.

Beispielsweise kann man die Reaktionspartner der allgemeinen Formel II mit einem Ueberschuß von 0,1 bis 0,3 Molaquivalenten einsetzen und dadurch einen geringere Zersetzung der Reaktionspartner der allgemeinen Formel III in einem wasserhaltigen Lösungsmittelgemisch erreichen. Der Ueberschuß der Reaktionspartner der allgemeinen Formel II laßt sich wegen der guten Löslichkeit in wäßrigen Mineralsauren beim Aufarbeiten des Reaktionsgemisches leicht entfernen.

Andererseits kann man aber auch mit Vorteil die Reaktionspartner der allgemeinen Formel III mit einem Ueberschuß von beispielsweise 0,1 bis 1,0 Molaquivalenten einsetzen. Dadurch werden die

Reaktionspartner der allgemeinen Formel II besser ausgenutzt und die als Nebenreaktion in wasserhaltigen Losungsmitteln ablaufende Zersetzung der Reaktionsteilnehmer der allgemeinen Formel III kompensiert. Da die im Ueberschuß zugesetzt a Verbindungen der allgemeinen Formel III sich in Wasser rasch in neutrale stickstoffhaltige Heterocyclen umwandeln, die sich leicht entfernen lassen, wird die Reinheit der antibiotica hierdurch kaum beeintrachtigt.

Die Menge der gegebenenfalls verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich ist oder nicht sinnvoll ist, werden vorzugsweise 2 Moläquivalente Base zugesetzt.

Die Aufarbeitung der Reaktionsansätze zur Herstellung der erfindungsgemäßen Verbindungen und ihrer Salze erfolgt durchweg in der bei diesen Körpern allgemein bekannten Art und Weise. Auch die Isolierung und Reinigung der erfindungsgemäßen Verbindungen sowie die Freisetzung der freien Säuren aus Salzen oder die Umwandlung der freien Säuren in Salze werden nach allgemein üblichen Methoden der organischen Chemie, welche jedem Fachmann geläufig sind, vorgenommen.

Alternativ sind die Verbindungen der Formel (I), bei denen R Niederalkoxy bedeutet auch durch Alkoxylierung der entsprechenden Wasserstoffderivate (R=H) erhältlich, wobei E eine geeignete Schutzgruppe bedeutet wie eine leicht entfernbare esterbildende Gruppe, eine Acetoxymethylgruppe, den kationischen Rest einer Base, vorzugsweise eines Alkali- oder Erdalkalimetallhydroxid oder Wasserstoff bezeichnet.

Bei diesem Verfahren setzt man eine $\beta$-Lactam-Verbindung der Formel (I), in der R Wasserstoff bedeutet, mit 2—10 Äquivalenten pro Äquivalent $\beta$-Lactam-Verbindung einer Base in Anwesenheit eines Überschusses eines Alkohols der Formel R'OH, in der R' Niederalkyl bezeichnet in einem inerten organischen Lösungsmittel um, fügt zwischen etwa 1 bis etwa 8 Äquivalente eines N-Halogenierungsmittels zu und isoliert die Verbindung der Formel (I) in der R Niederalkoxy bedeutet, gegebenenfalls nach vorheriger Abspaltung der Säureschutzgruppe, Überführung in ein Salz oder einen pharmazeutisch verwendbare Ester isoliert.

Bei diesem erfindungsgemäßen Verfahren werden als N-Halogenierungsmittel, vorzugsweise positives Chlor übertragende Verbindungen, wie t-Butylhypochlorit oder Chloracetamid verwendet.

Als Basen eignen sich komplexe und einfache, vorzugsweise jedoch einfache, Alkali- und Erdalkalihydride, metallorganische Verbindungen sowie Grignard-Verbindungen. Als Beispiele seien genannt: Lithiumhydrid, Natriumhydrid, Butyl-Lithium, Phenyl-Lithium, Alkyl-Magnesiumbromid, beispielsweise Methyl-Magnesiumbromid, oder andere bekannte Säurebindemittel wie Alkali- und Erdalkali-alkoholate oder -carbonate, Alkali- und Erdalkali-bicarbonate oder -oxide wie beispielsweise Natriumcarbonat oder andere Säurebindemittel wie Borax oder offenkettige oder cyclische organische Basen wie Trialkyl- oder Aralkylamine oder cyclische Amidine wie 2,3,4,6,7,8 - Hexahydro - pyrrolo[1,2 - *a*]pyrimidin (DBN) oder 2,3,4,6,7,8,9,10 - Octahydro - pyrimido[1,2 - *a*]azepin (DBU).

Als Lösungsmittel eignen sich beispielsweise offenkettige oder cyclische Äther, aliphatische und aromatische Kohlenwasserstoffe oder Halogenkohlenwasserstoffe oder die Alkohole R'OH. Besonders geeignet ist Tetrahydrofuran.

Die Reaktionstemperaturen sind möglichst unter 0°C zu halten, vorzugsweise zwischen —70°C und —45°C.

Die Verbindungen der allgemeinen Formel I sind in Form der freien Säure sowohl kristallin wie amorph und sowohl wasserfrei wie in verschiedenen Hydratformen in gleicher Weise antibakteriell wirksam. Ebenfalls sind die Verbindungen der allgemeinen Formel I in Form ihrer Salze, z.B. Natriumsalze, sowohl kristallin wie amorph und sowohl wasserfrei wie wasserhaltig, beispielsweise als Hydrat, in gleicher Wiese antibakteriell wirksam.

Als neue Wirkstoff seien beispielhaft genannt (Formeln (IV) und (V)

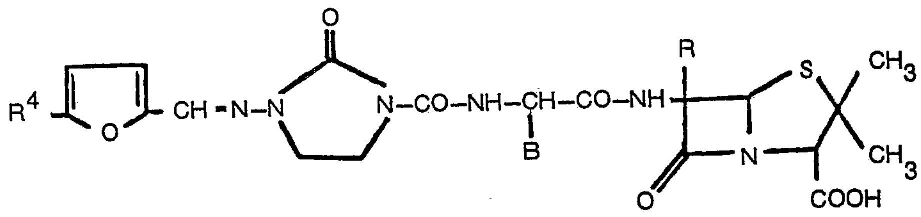

(IV)

| $R^4$ | B | R |
|---|---|---|
| H | Furyl-2 | H |
| $CH_3$ | " | " |
| Cl | " | " |
| Br | " | " |
| H | " | $CH_3O$ |
| H | Furyl-3 | H |
| $CH_3$ | " | " |
| Cl | " | " |
| Br | " | " |
| H | " | $CH_3O$ |
| H | Thienyl-2 | H |
| H | Thienyl-2 | $OCH_3$ |
| H | Thienyl-3 | H |
| H | Thienyl-3 | $OCH_3$ |
| H | 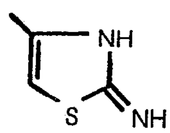 | H |
| H | " | $OCH_3$ |

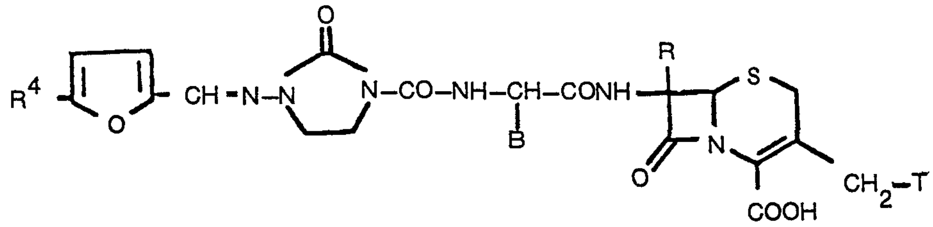

(V)

| $R_4$ | B | R | T |
|---|---|---|---|
| H | Furyl-2 | H | OAc |
| $CH_3$ | " | " | " |
| Cl | " | " | " |
| Br | " | " | " |
| H | Furyl-2 | $OCH_3$ | OAc |
| H | " | H | 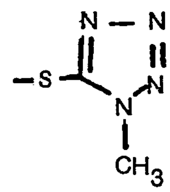 |
| H | Furyl-3 | H | OAc |
| $CH_3$ | " | " | " |
| Cl | " | " | " |
| Br | " | " | " |
| H | " | $OCH_3$ | " |
| H | Thienyl-2 | H | OAc |
| H | " | $OCH_3$ | " |
| H | Thienyl-3 | H | " |
| H | " | $OCH_3$ | " |
| H | 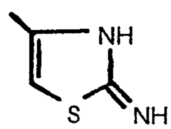 | H | OAc |
| H | " | $OCH_3$ | OAc |

Die erfindungsgemäßen Wirkstoffe weisen bei geringer Toxizität eine starke und breite antimikrobielle Wirksambeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemaßen Wirkstoffe sind gegen ein sehr breites Spektrum von Mikroorganismen wirksam. Mit ihrere Hilfe können gramnegative und grampositive Bakterien und bakterienahnliche Mikroorganismen bekampft sowie die durch diese Erreger hervorgerufenen Erkrankungen verhindert, gebessert und/oder geheilt werden.

Besonders wirksam sind die erfindungsgemaßen Wirkstoffe gegen Bakterien und bakterienähnliche Mikroorganismen. Sie und daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. aerogenes und Gaffkya tetragena (Staph. = Staphylococcus);

Lactobacteriaceae, wie Streptokokken, z.B. Streptococcus pyogenes, $\alpha$-bzw. $\beta$-hämolysierende Streptokokken, nicht ($\gamma$-)-hämolysierende Streptokokken, Str. viridans, Str. faecalis (Enterokokken), Str. agalactiae, Str. lactis, Str. equi, Str. anaerobis und Diplococcus pneumoniae (Pneumokokken) (Str. = Streptococcus);

Neissericeae, wie Neisserien, z.B. Neisseria gonorrhoeae (Gonokokken), N.meningitidis (Meningokokken), N.catarrhalis und N.flava (N. = Neisseria);

Corynebacteriaceae, wie Corynebakterien, z.B. Corynebacterium diphtheriae, C.pyogenes, C.-diphtheroides, C.acnes, C.parvum, C.bovis, C.renale, C.ovis, C.murisepticum.

Mycobacteriaceae, wie Erreger von Mykobakteriosen, z.B. Mycobacterium tuberculosis, M.bovis, M.avium, sogenannte atypische Mykobakterien der Runyon-Gruppen, I, II, III und IV, M.leprae (M. = Mycobacterium);

Enterobacteriaceae, wie Escherichiae-Bakterien der Coli-Gruppe: Escherichia-Bakterien, z.B. Escherichia coli, Enterobacter-Bakterien, z.B. E.aerogenes, E.cloacae, Klebsiella-Bakterien, z.B. K.pneumoniae, K.pneumoniae, K.ozaenae, Erwiniae, z.B. Erwinia spec., Serratia, z.B. Serratia marcescens (E. = Enterobacter) (K. = Klebsiella), Proteae-Bakterien der Proteus-Gruppe: Proteus, z.B. Proteus vulgaris, Pr.morganii, Pr.rettgeri, Pr.mirabilis, Providencia, z.B. Providencia sp. (Pr. = Proteus), Salmonelleae: Salmonella-Bakterien, z.B. Salmonella paratyphi A und B. S.typhi, S.enteritidis, S.-cholerae suis, S.typhimurium (S. = Salmonella, Shigella-Bakterien, z.B. Shigella dysenteriae, Sh.ambigua, Sh.flexneri, Sh.boydii, Sh.sonnei (Sh. = Shigella);

Pseudomonadaceae, wie Pseudomonas-Bakterien, z.B. Pseudomonas aeruginosa, Ps.pseudomallei (Ps. = Pseudomonas), Aeromonas-Bakterien, z.B. Aeromonas liquefaciens, A.hydrophila (A. = Aeromonas);

Parvobacteriaceae, wie Pasteurella-Bakterien, z.B. Pasteurella multocida, Past.pestis (Yersinia), Past.pseudotuberculosis,Haemophilus-Bakterien, z.B. Haemophilus influenzae, H.ducreyi, H.suis, H.-canis, H.aegypitcus (H = Haemophilus), Bordetella-Bakterien, z.B. B.bronchiseptica (B. = Bordetella).

Bacteroidaceae, wie Bacteroides-Bakterien, z.B. Bacteroides fragilis, B.serpens (B. = Bacteroides), Fusiforme-Bakterien, z.B. Fusobacterium fusiforme, Sphaerophorus-Bakterien, z.B.Sphaerophorus necrophorus, Sph-necroticus, Sph.pyrogenes (Sph. = Sphaerophorus);

Bacillaceae, wie aerobe Sporenbildner, z.B. Bacillus anthracis (B.subtilis, B.cereus) B. = Bacillus), Anaerobe Sporenbildner-Chlostridien, z.B. Clostridium perfringens, Cl.septicium, Cl.oedematien, Cl.histolyticum, Cl.tetani, Cl.botulinum (Cl. = Clostridium).

Die obige Aufzahlung von Erregern ist lediglich beispielhaft und keineswegs beschrankend aufzufassen.

Als Krankheiten, die durch die erfindungsgemaßen Wirkstoffe verhindert, gebessert und/oder geheilt werden können, seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehoren pharmazeutische Zubereitungen, die neben nichttoxischen, inerten pharmazeutisch geeigneten Tragerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Tragerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker,

Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Ueberzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Hintettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien konnen neben dem oder den Wirkstoffen die üblichen wasserloslichen oder wasserunloslichen Trägerstoffe enthalten, z.B. Polyathylenglykole, Fette, z.B. Kakaofett und hohere Ester (z.B. $C_{14}$-Alkohol mit $C_{16}$-Fettsaure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Starke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen konnen neben dem oder den Wirkstoffen die üblichen Trägerstoffe wie Lösungsmittel, Losungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatol, Erdnußol, Maiskeimol, Olivenöl, Ricinusöl und Sesamol, Glycerin, Glycerinformal, Tetrahydrofurfurylalkohol, Polyathylenglykole und Fettsaureester des Sorbitans oder Gemische dieser Stoffe enthalten.

Zur parenteralen applikation konnen die Lösungen und Emulsionen auch in steriler und blutisotonischer Form vorliegen.

Suspensionen konnen neben dem oder den Wirkstoffen die üblichen Trägerstoffe wie flüssige Verdünnungsmittel, z.B. Wasser, Aethylalkohol, Propylenglykol, Suspendiermittel, z.B. athoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Tragerstoffen.

Zur vorliegenden Erfindung gehört auch die Verwendung der erfindungsgemaßen Wirkstoffe sowie von pharmazeutischen Zubereitungen, die einen oder mehrere erfindungsgemäße Wirkstoffe enthalten, in der Human- und Veterinarmedizin zur Verhutung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise oral oder parenteral wie intravenös oder intramuskular appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 5 bis etwa 1000, vorzugsweise 20 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 10 bis 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fallen die oben angeführte Wirkstoffmenge überschritten werden

muß. Die Festlegung der jeweils erforderlichen optimalen Dosierung und Applikationsart der Wirkstoff kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die neuen Penicilline und Cephalosporine zeichnen sich durch starke antibakterielle Wirkungen, die in vivo und in vitro geprüft wurden, aus.

Die erfindungsgemäßen Penicilline und Cephalosporine können zum Zwecke der Erweiterung des Wirkungsspektrums und um eine Wirkungssteigerung speziell bei β-lactamasebildenden Bakterien zu erzielen mit anderen antimikrobiellen Wirkstoffen z.B. mit Penicillinen, die besonders penicillinasefest sind, kombiniert werden. Eine solche Kombination wäre z.B. die mit Oxacillin oder Dicycloxacillin.

Die erfindungsgemäßen Penicilline und Cephalosporine können zum Zweck der Erweiterung des Wirkungsspektrums und um eine Wirkungssteigerung zu erreichen auch mit Aminoglykosidantibiotica, wie z.B. Gentamicin, Kanamicin, Sisomicin, Amikacin oder Tobramicin, kombiniert werden.

Die Wirksamkeit der erfindungsgemäßen β-Lactamantibiotika kann durch die folgenden in vitro- und in vivo-Versuche beispielhaft demonstriert werden:

1. *In vitro-Versuche*

Die Beispiele 2.3 und 3.1, welche als typische Vertreter der erfindungsgemäßen Verbindungen betrachtet werden können, wurden mit Müller-Hinton-Nährbrühe unter Zusatz von 0,1% Glucose auf einen Gehalt von 100 μg/ml verdünnt. In der Nährlösung befanden sich jeweils $1 \times 10^5$ bis $2 \times 10^5$ Bakterien pro Milliliter. Die Röhrchen mit diesem Ansatz wurden jeweils 24 Stunden bebrütet und anschließend wurde der Trübungsgrad bestimmt. Trübungsfreiheit gibt Wirkung an. Bei der Dosierung von 100 μg/ml waren die folgenden Bakterienkulturen trübungsfrei (sp. = species):

Klebsiella pneumoniae; Enterobacter aerogenes sp.; Providencia; Serratia marcescens; E.coli BE; Salmonella sp.; Shigella sp.; Proteus, indolnegativ und indolpositiv; Pasteurella pseudotuberculosis; Brucella sp.; Haemophilus influenzae; Bordetella bronchiseptica; Staphylococcus aureus 133; Neisseria catarrhalis sp.; Diplococcus pneumoniae sp.; Streptococcus pyogenes W.; Enterococcus sp.; Lactobacillus sp.; Corynebacterium diphteriae gravis; Corynebacterium pyogenes M; Clostridium tetani; Pseudomonas aeruginosa sp.

Bei den NMR-Spektren der erfindungsgemäßen Verbindungen bedeuten die Bezeichnungen:

| | | |
|---|---|---|
| s | = | Singulett |
| d | = | Dublett |
| m | = | Multiplett |
| AB | = | AB-System |

Erläuterung der in den Beispielen verwendeten Abkürzungen:

| | | |
|---|---|---|
| Vol. | = | Volumen |
| Gew.-Tle. | = | Gewichtsteile |
| Vol.-Tle. | = | Volumenteile |
| Std. | = | Stunden |
| Stde. | = | Stunde |
| THF. | = | Tetrahydrofuran |
| Äther | = | Diäthyläther |
| Essigester | = | Essigsäureäthylester |
| Raumtemperatur | = | ca. 20°C |
| abs. | = | absolut |

Zers.-p = Zersetzungspunkt

Die Ausbeuteangaben in % bedeuten Ausbeuten in % der Theorie.

Beispiel 1

*2-t-Butoxycarbonylamino-furfurylessigsäure*

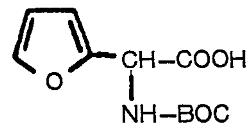

5,0 Gew.Tle. 2-Amino-furfurylessigsäure (Röhm und Haas), Neth.Appl.66,07754) in 100 Vol.Tln. 80-proz. wäßrigem Dioxan werden mit 4N Natronlauge auf pH 8 gebracht. Man gibt 8,0 Gew.Tle. 2-(t-Butoxycarbonyloxyimino)-2-phenylacetonitril zu und erwärmt 2 Stdn. auf 70°. Während dieser Zeit wird durch Zugabe von 4N Natronlauge der obige pH aufrechterhalten. Danach werden 80 Vol.Tle.-Wasser zugegeben, das Dioxan abgezogen und mehrere Male mit Essigester extrahiert. Die wäßrige Phase wird mit 10-proz. Zitronensäure auf pH 4 gebracht, mit Kochsalz gesättigt und mit Essigester ausgeschüttelt. Die Essigesterphasen werden über Natriumsulfat getrocknet und eingeengt. Der Rückstand wird aus wenig abs. Tetrachlorkohlenstoff umkristallisiert. Man erhält 4,3 Gew.Tle. 2-t-Butoxycarbonylamino-furfurylessigsäure vom Fp. 99—101°.

IR(KBr): 3357, 1720, 1686, 1513, 1154, 747 $cm^{-1}$.
NMR($CD_3OD$): seudo-s 7.42(1H), pseudo-s (6.35(2H), s 5.30(1H), s 1.45(9H) ppm ($\delta$).

Beispiel 2.1

*7-[α-(t-Butyloxycarbonylamino)-furfurylacetamido]-3-acetoxymethyl-ceph-3-em-4-carbonsäure*

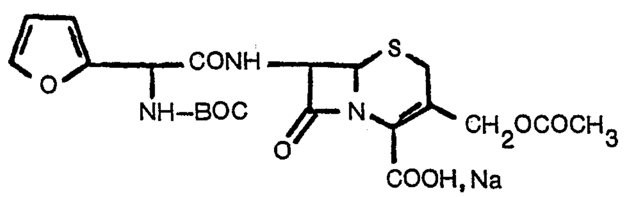

Zu einer Lösung von 3,7 Gew.Tln. 2-t-Butoxycarbonylaminofurfurylessigsäure in 60 Vol.Tln. abs. THF werden 1,7 Gew.Tle. Triäthylamin sowie 0,1 Gew.Tle. N-Methylmorpholin gegeben. Es wird auf −25° gekühlt, eine Lösung von 1,8 Gew.Tln. Chlorameisensäureäthylester in 10 Vol.Tln. abs. THF zugefügt und 15 Min. bei dieser Temperatur gerührt. Dann wird eine auf −20° vorgekühlte Lösung von silylierter 7-Amino-cephalosporansäure (7—ACS) in 40 Vol.Tln. abs. THF (aus 5,0 Gew.Tln. 7—ACS und 5,6 Gew.Tln. Bistrimethylsilylacetamid hergestellt) zugegeben, 10 Min. bei −20° gerührt und auf RT kommen gelassen. Es werden 80 Vol.Tle. Eiswasser zugegeben, auf pH 7,3 gestellt und das THF abgezogen. Die wäßrige Phase wird mit Essigester extrahiert, bei 0—5° angesäuert und erneut mit Essigester extrahiert. Die letzteren Essigesterauszüge werden über Magnesiumsulfat getrocknet und eingeengt. Man erhält das Produkt (7,5 Gew.Tle.) als gelben Schaum, der direkt weiter verarbeitet wird.

Beispiel 2.2

*7-[(α-Amino)-furfurylacetamido]-3-acetoxymethyl-ceph-3-em-4-carbonsäure, Trifluoracetat*

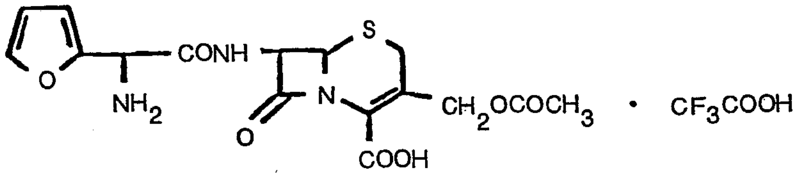

7,5 Gew.Tle. 7 - [α - (t - Butyloxycarbonylamino) - furfurylacetamido] - 3 - acetoxymethyl - ceph - 3 - em - 4 - carbonsäure werden in 30 Vol.Tln. eiskalter Trifluoressigsäure gelöst und 15 Min. bei 0° gerührt. Die Lösung wird in ein Gemisch aus 120 Vol.Tln. Petroläther und 60 Vol.Tln. Aether gegossen, wobei das Produkt als Trifluoracetat ausfällt. Es wird abgesaugt, mit Aether gewaschen und über Phosphorpentoxid und Kaliumhydroxid getrocknet. 5,8 Gew.Tle. vom Zers. p. 115—123°.

Beispiel 2.3

*Natrium-7-{α[(2-oxo-3-furfurylidenamino-imidazolidin-1-yl)-carbonylamino]-furfurylacetamido}-3-acetoxymethyl-ceph-3-em-4-carboxylat*

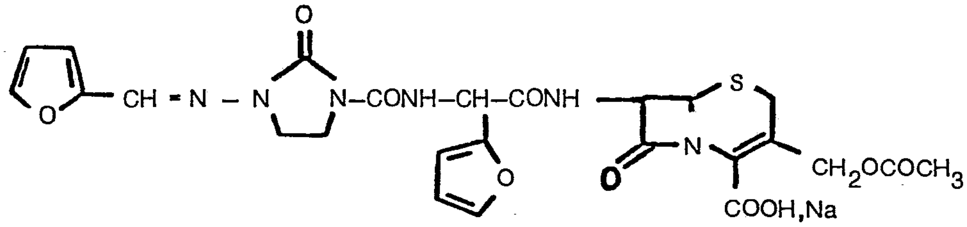

Eine Lösung von 5,8 Gew.Tln. 7 - [(α - Amino) - furfurylacetamido] - 3 - acetoxymethyl - ceph - 3 - em - 4 - carbonsäure als Trifluoracetat in 100 Vol.Tln. 80-proz. wäßrigem THF wird auf 5° gekühlt und mit 1N Natronlauge auf pH 8 gebracht. 2,8 Gew.Tle. 1-Chlorcarbonyl-2-oxo-3-furfurylidenamino-imidazol werden portionsweise zugefügt, währenddessen der pH mit 0,5 N Natronlauge auf 7,5 gehalten wird. Wenn der pH-Wert konstant ist, werden 100 Vol.Tle. Wasser zugegeben und das THF abgezogen. Die wäßrige Lösung wird mit Essigester extrahiert, auf 5° gekühlt, mit 150 Vol.Tln. Essigester überschichtet und mit 1N Salzsäure angesäuert, wobei die Produkt-Säure ausfällt (3,5 Gew.Tle.). Sie wird in 35 Vol.Tln. Wasser suspendiert, mit 0,5 N Natronlauge gelöst und diese Lösung lyophilisiert. Man erhält 3,0 Gew.Tle. Natriumsalz vom Zers. p. 170—180°.

IR (KBr): 3420, 1765, 1725, 1675, 1605, 1415, 1235 $cm^{-1}$.
NMR($CD_3OD$):
s 7.75(1H), pseudo-s 7.64(1H), pseudo-s 7.50(1H), d 6.89(1H), m um 6.5(3H), m um 5.65(2H), m um 5.0(teilweise verdeckt vom Signal der austauschbaren Protonen), s(breit) 3.93(4H), AB bei 3.4 und 3.6 (verdeckt vom Lösungsmittelsignal), s 2.02(3H) ppm (δ).

$C_{25}H_{23}N_6NO_{10}S \cdot 2{,}5\ H_2O$

| | | | | |
|---|---|---|---|---|
| ber: | C 44,95 | H 4,23 | N 12,59 | S 4,80 |
| gef.: | C 44,9 | H 4,1 | N 12,8 | S 4,8 |

Beispiel 3.1

*Natrium-6-{α-[(2-oxo-3-furfurylidenamino-imidazolidin-1-yl)-carbonylamino]-furfurylacetamido}-penicillanat*

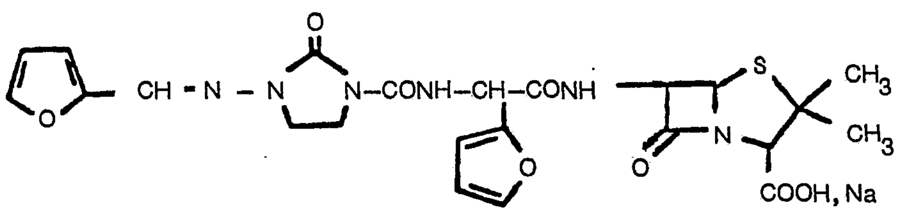

2,4 Gew.Tle. α-Amino-furfurylacetamido-penicillansäure (Beecham, USP 3.120.514) und 2,4 Gew.Tle. 1-Chlorcarbonyl-2-oxo-3-furfurylidenamino-imidazol werden wie im Beispiel 2.3 umgesetzt und aufgearbeitet. Man erhält 3,3 Gew.Tle. vom Zers. p. 180—185° mit einem β-Lactamgehalt von 82%.

IR(KBr): 1760, 1715, 1660, 1605 $cm^{-1}$.
NMR($CD_3OD$):
s 7.72(1H), d 7.70(1H), pseudo-s 7.50(1H), d 6.85(1H), m um 6.60(3H), s 5.55(1H), AB 5.38 und 5.42(2H), s 4.12(1H), s(breit) 3.80(4H), s 1.53(3H), s 1.48(3H) ppm (δ).

Beispiel 4

*Natrium-6-{α-[(2-oxo-3-furfurylidenamino-imidazolidin-1-yl)-carbonylamino]-thienyl-2-acetamido}-penicillanat*

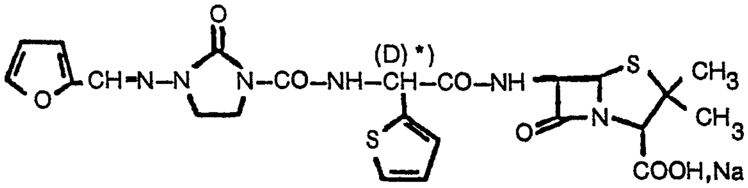

0,6 Gew.-Teile α-Amino-thienyl-(2)-acetamido-penicillansäure (M. Hatanaka, T. Ishimaru, J. Medicinal Chemistry 1973, *16*, S. 978—84) werdenin 40 Vol.-Tln. 80-%igem wäßrigen Tetrahydrofuran durch Zusatz der gerade eben nötigen Menge Triäthylamin bei pH 7,5—8,0 gelöst. Anschließend wird mit 0,42 Gew.-Tln. 1-Chlorcarbonyl-2-oxo-3-furfurylidenamino-imidazol in der im Beispiel 2.3 beschriebenen Weise umgesetzt und aufgearbeitet.

Ausbeute: 0,7 Gew.-Tle.
β-Lactamgehalt: 85% (Substanz enthält ca. 6% β-Lactamoffenes Produkt)

IR (Nujol; Carbonylbereich):
1760 (Schulter), 1750, 1715, 1655, 1595, 1520—10 $cm^{-1}$.
NMR ($CD_3OD/D_2O$):
7,7—6,9 m (5H, Thiophen, Furan, —CH=N—), 6,8 d (1H, Furan), 6,5 m (1H, Furan), 5,8 s (1H, >N—CH—CO—), 5,5 pseudo-s (2H, 5.6-H), 4,2 s (1H, 3-H), 3,9—3,6 s, breit (4H, $CH_2CH_2$), 1,55 s (3H) und 1,45 s (3H) ppm (δ).

Nach dem NMR-Spektrum enthält die Substanz etwa 0,08 Moläquivalente Natrium-2-äthylhexanoat und etwa 3,2 Moläquivalente Wasser.

Beispiel 5.1

*2-(1,3,5-Trimethylpyrazol-4-yl)-2-aminoessigsäure*

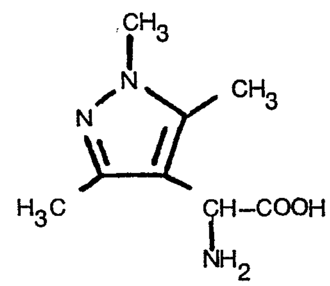

Eine Mischung aus 13,8 Gew.-Tln. 1,3,5-Trimethylpyrazol-4-aldehyd, 5 Gew.Tle. Natriumcyanid, 5,9 Gew.-Tle. Ammoniumchlorid, 13,4 Vol.Tle. konzentrierte Ammoniaklösung, 30 Vol.Tle. Wasser und 30 Vol.Tle. Aethanol werden 5 Stdn. bei 60°C in einem verschlossenen Kolben unter gelegentlichem Umschwenken stehengelassen.

Dann gibt man die Reaktionslösung in 80 Vol.Tle. eiskalte konzentrierte Salzsäure, spült mit Wasser nach und leitet 50—60 g HCl-Gas bei 0—5°C in die Lösung ein, die man anschließend über Nacht stehenlaßt (Abzug). Nach Verdünnung mit 100 Vol.Tln. Wasser, 2,5 Stdn. Rückflußkochen und Einengen wird der Rückstand zweimal mit Wasser in einer Porzellanschale (zur Entfernung von HCl) abgeraucht.

Schließlich nimmt man in Aethanol auf, filtriert vom Ungelösten ab, engt zur Trockne ein, nimmt wieder in Wasser auf und verrührt mit ca. 50 g saurem Ionenaustauscher (Dowex der Fa. Serva, Heidelberg) (5 Stunden).

Dann saugt man den beladenen Ionenaustauscher ab, wäscht gut mit Wasser nach und eluiert schließlich mit Ammonium-carbonatlösung. Diese Lösung wird zur Trockne eingeengt. Der Rückstand wird mit Aethanol gewaschen und aus den Mutterlaugen 7,4 Gew.Tle. (40%) der gewünschten Aminosäure erhalten.

60 MHz-NMR-Spektrum ($D_2O$):

*)Aufgrund des NMR-Spektrums und der guten Wirksamkeit muß man annehmen, daß die D-Form vorliegt. Da vom Racemat der α-Amino-thienylessigsäure ausgegangen wurde, muß auf der Stufe des α-Amino-thienylmethylpenicillins eine Diastereomerentrennung eingetreten sein.

2,05 ppm s (3H), 2,15 s (3H), 3,6 s (3H), 4,8 (Wasser), 4,9 s (1H).

Dünnschichtchromatographisch einheitlich $R_f = 0$.
(Laufmittel: 200 ml n-Butylacetat, 36 ml n-Butanol, 100 ml Essigsäure (behandelt mit 150 ml Phosphatpuffer pH 6)).

Beispiel 5.2

*2-(1,3,5-Trimethylpyrazol)-4-yl)-2-tert.-butoxycarbonylaminoessigsäure*

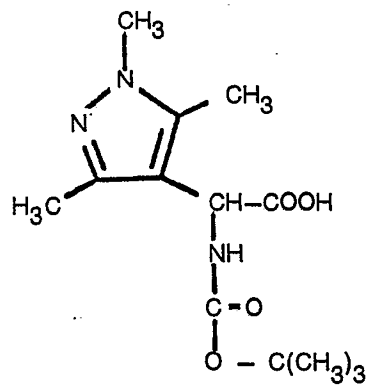

Eine Mischung aus 16,1 Gew.-Tln. Aminosäure aus Beispiel 7.1.
3.52 Gew.-Tln. Natriumhydroxid
18 Vol.-Tln. t-Butanol
9 Vol.-Tln. Wasser

wird bei 0°C mit 20,3 Gew.-Tln. Di-t-butyl-dicarbonat langsam versetzt, noch weitere 18 Vol.-Tle. t-Butanol hinzugefügt und 14 Stunden bei 25°C, dann 12 Stunden bei 50°C nachgerührt. Man verdünnt mit 50 Vol.Tln. Wasser, wäscht je 3mal mit n-Pentan und Äther und säuert auf pH 2,4 an. Nach Extraktion mit Essigsäureäthylester (4 ml), Trocknen über $Na_2SO_4$ und Einengen wird im Hochvakuum getrocknet.

60 $MH_2$-NMR-Spektrum ($CDCl_3$):
1,4 ppm s (9H), 2,4 s (3H), 2,45 s (3H), 3,7 s (3H), 5,1 d (1H), 5,8 d (1H), 12,6 s (1H).

Ausbeute: 15 Gew.-Tle. (63%) 2-(1,3,5-Trimethylpyrazol-4-yl)-2-tert.-butoxycarbonylaminoessigsäure.

Beispiel 5.3

*7-[2-(1,3,5-Trimethylpyrazol-4-yl)-2-t-butoxycarbonylamino-acetamido]-cephalosporansäure*

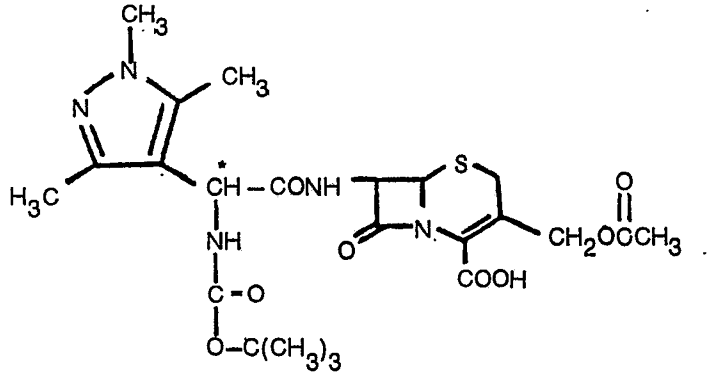

*(D,L-Gemisch)

5,36 Gew.-Tle. der Verbindung aus Beispiel 5.2 werden in 150 Vol.-Tln. Tetrahydrofuran, 2 Gew.-Tln. Triäthylamin gelöst, bei −15°C mit 2,75 Gew.-Tln. Chlorameisensäure-iso-butylester versetzt und 1,5 Std. bei −10°C nachgerührt (Lösung A).

Lösung B:

5,44 Gew.-Tle. 7-Aminocephalosporansäure werden in 100 Vol.-Tln. 80%igem wäßrigen Tetrahydrofuran und 2 Gew.-Tln. Triäthylamin gelöst und auf —10°C gekühlt. Hierzu tropft man Lösung A bei —10°C. Es wird 1,5 Std. bei —10°C und noch weitere 2 Stdn., wobei man auf Raumtemperatur kommen läßt, nachgerührt.

Die Reaktionslösung wird in 100 Vol.-Tle. Wasser gegeben, mit Essigester gewaschen, angesäuert (pH 1,8), extrahiert mit Essigester und diese Essigesterlösung mit gesättigter NaCl-Lösung gewaschen. Nach dem Trocknen über $MgSO_4$ wird eingeengt und der Rückstand im Hochvakuum getrocknet.

Ausbeute: 7,5 Gew.-Tle. (70%) der oben bezeichneten Verbindung.

Dünnschichtchromatogram einheitlich $R_f$ —Wert 0,3 (Laufmittelgemisch wie in Beispiel 7.1)

100 MHz-NMR-Spektrum ($CDCl_3$):

1,4 ppm s (9H), 2,1 s (3H) 2,3 m (6H), 3,4 a,b(2H), 3,7 m(3H), 4,95 a,b (2H), 5,05 d (1H) (I=3Hz), 5,3 d (1H) (I=3Hz), 5,8 d (1H), 7,3 d (1H), 10,8 s (1H).

Beispiel 5.4

*7-[2-(1,3,5-Trimethylpyrazol-4-yl)-2-amino]-acetamidocephalosporansäure*

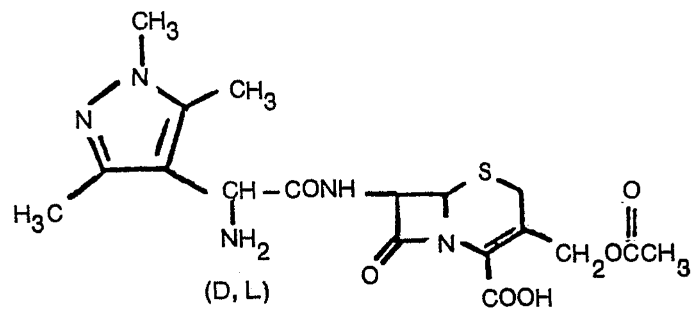

(D, L)

12,2 Vol.-Tle. Trifluoressigsäure und 1,4 Vol.-Tle. Anisol werden auf 0°C gekühlt und unter Rühren 4 Gew.-Tle. der Verbindung aus Beispiel 5.3 zugegeben.

Anschließend wird die Trifluoressigsäure weitgehend abdestilliert (Vorlage auf —70°C gekühlt, Hochvakuum) und der Rückstand in 10 Vol.-Tln. Methylenchlorid gelöst und 20 Min. gerührt. Dann gibt man 100 Vol.-Teile Äther/Ligroin 1:1 zu und rührt weitere 30 Min. nach, saugt den Neiderschlag ab und nimmt ihn in Wasser auf.

Nach Überschichten mit Essigester/Äther stellt man mit Amberlite (flüssiger Ionenaustauscher, Chlorid als Gegenion) auf pH 7, trennt die Phasen und wäscht die wäßrige Phase sehr gründlich mit Äther. Die wäßrige Phase wird gefriergetrocknet.

Ausbeute: 3 Gew.-Tle. 7-[2-(1,3,5-Trimethylpyrazol-4-yl)-2-amino]-acetamidocephalosporansäure.

Dünnschichtchromatogram (Laufmittel siehe Beispiel 5.1) ein Substanzfleck am Startpunkt.

IR(KBr) 3422, 2923, 1763, 1689, 1599, 1383, 1235, 1064, 1026, 739.

100 MHz-NMR-Spektrum ($D_2O$):

2,1 ppm 2 s (Abstand 0,5 Hz) 3H (Verhältnis 1:1), 2,2 m (3H) 2,3 m (3H), 3,4u 3,6 a,b (2H), 3,7s (3H), 4,7 s (4 ausgetauschte H + 1 x $H_2O$), 5,15 m (3H), 5,7 m (1H).

Beispiel 5.5

*Natrium-7-{2-(1,3,5-Trimethylpyrazol-4-yl)-2-[(2-oxo-3-furfurylidenaminoimidazolidin-1-yl)-carbonylamino]-acetamido}-cephalosporanat*

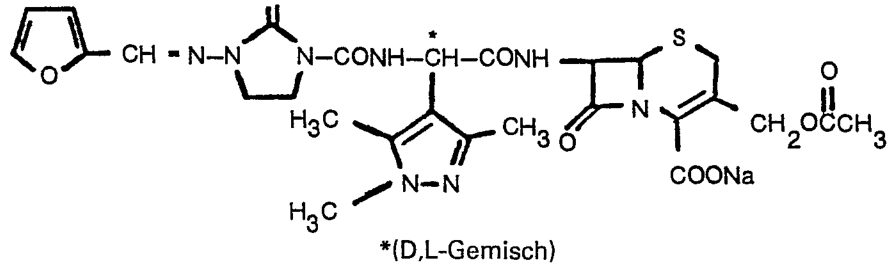

*(D,L-Gemisch)

1 Gew.Tl. des vorstehend beschriebenen Cephalosporins wird in 80 Vol.Tln. 80%-igem wäßrigen Tetrahydrofuran gelöst und 0,53 Gew.Tle und 0,53 Gew.Tle. 1-Chlorcarbonyl-2-oxo-3-furfurylidenamino-imidazol zugesetzt, wobei man mit NaOH auf pH 7—8 hält; Temperatur 5°C.
Aufarbeitung wie in Beispiel 2.3. beschrieben.
Ausbeute: 1,2 Gew.Tle. (78%) der oben bezeichneten Verbindung.
IR-Spektrum (Nujol) $\beta$-Lactambande bei 1765.

Dünnschichtchromatogramm (Laufmittel wie Beispiel 5.1.) $R_f$-Wert: 0,2 (einheitlich).

100 MHz-NMR-Spektrum ($CD_3OD+D_2O$):
2,15 ppm s (3H), 2,3 m (3H), 2,45 m (3H), 3,4 a,b (verdeckt durch Lösungsmittelsignal und folgende Signale) (2H), 3,8s (3H), 4,0 s (breit) (4H), 4,9—5,2 m (teilweise verdeckt durch ausgetauschte Wasserstoffe) (4H), 5,5 d (J=0,5Hz) (1H), 6,65 q (1H), 7,0 d (1H), 7,8 d (1H), 7,85 s (1H).

Beispiel 6

*Natrium-6α-methoxy-6β-{D-2-[(2-oxo-3-furfurylidenaminoimidazolidin-1-yl)-carbonylamino]-thienyl-2-acetamido}-penicillinat*

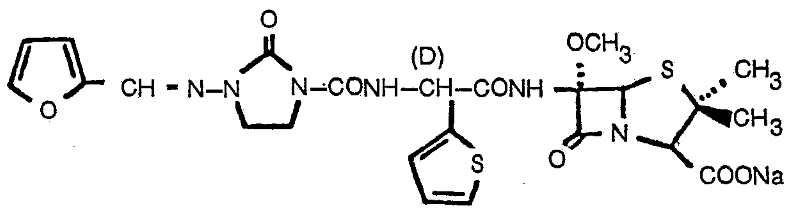

1,9 Gew.Tle. 6-{D-2-[(2-oxo-3-furfurylidenamino-imidazolidin-1-yl)-carbonylamino]-thienyl-2-acetamido}-penicillinsäure in 40 Vol.Tln. absolutem Tetrahydrofuran werden bei —80°C mit 0.067 Gew.Tln. Lithiumhydrid in 20 ml Methanol versetzt; anschließend gibt man 0,4 Gew.Tle. t-Butylhypochlorit zu und rührt 2 Stdn. bei —60°C nach.

Die Reaktionslösung wird dann in eine 10%-ige Ammoniumchlorid-Lösung gegeben und hält den pH-Wert unter weiterer Zugabe von verdünnter Salzsäure bei 7. Das Tetrahydrofuran wird im Vakuum abdestilliert, und die wäßrige Phase mit Essigester gewaschen. Dann gibt man 10 Vol.Tle. Aceton zu und stellt den pH-Wert unter Rühren langsam mit verdünnter Salzsäure auf 4. Die Penicillansäure kristallisiert langsam aus. Den Niederschlag saugt man ab und löst ihn in Wasser unter Zugabe von 1N Natronlauge bei pH-Wert 7. Die Lösung wird schließlich lyophilisiert.

Man erhält 1,8 Gew.Tle. (86%) der oben bezeichneten Verbindung.

Dünnschichtchromatogramm: $R_f$-Wert 0,6 einheitlich.

IR-Spektrum (KBr):
3436, 1762, 1726, 1674, 1604, 1529, 1476, 1413, 1270, 1235, 1136, 740.

100 MHz-NMR-Spektrum ($CD_3OD$):
1,3 ppm s (3H), 1,5 s (3H), 3,6 s (3H), 4,0 s (breit) (4H), 4,3 s (1H), 5,65 s (1H), 6,0 d (1H), 6,65 q (1H), 7,0 d (1H), 7,1—7,5 m (5H), 7,75 d (1H), 7,8 s (1H).

Beispiel 7.1

*2-(2-tert.-Butoxycarbonylimino-4-thiazolin-4-yl)-2-methoxyiminoessigsäureäthylester (syn-Form):*

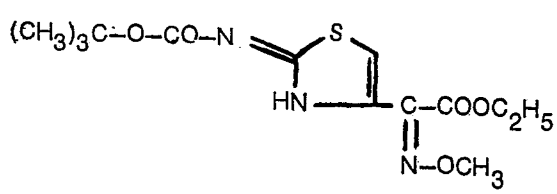

Zu der auf 40—45°C erwärmten und gerührten Mischung von 50 g 2-(2-Aminothiazol-4-yl)-2-methoxyiminoessigsäureäthylester (syn-Form) und 150 ml tert.-Butanol wird 65 g Di-tert.-Butylpyrocarbonat zugetropft, anschließend 30 Minuten bei 40°C nachgerührt und dann das Lösungsmittel im Vakuum entfernt. Die Substanz wird dann in Aether gelöst, diese Lösung mit verdünnter Salzsäure gewaschen, wobei 13 g unumgesetztes Ausgangsmaterial ausfällt. Es wird abgesaugt, das Filtrat mit etwas Petrolaether versetzt, worauf noch 4,5 g Ausgangsmaterial ausfällt. Das Filtrat dieser

Fällung wird dann wieder im Vakuum eingedampft, der Rückstand in einer Mischung von Petrolaether und Aceton (9:1) gelöst und über eine Kieselgelsäule chromatographiert. Die gesuchte Verbindung wird als zweite Substanz eluiert. Schmelzpunkt 122—24°C

NMR (60 MHz; δ in $CCl_4$) 7,05 (s; Thiazolin 5-H)

Beispiel 7.2

*2-(2-tert.-Butoxycarbonylimino-4-thiazolin-4-yl)-2-aminoessigsäureäthylester-hydrochlorid:*

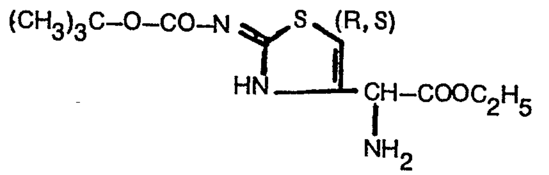 HCl

Zur eisgekühlten Mischung von 7,0 g einer nach Beispiel 7.1 dargestellten Substanz und 80 ml 90 %iger wäßriger Essigsäure gibt man 5,6 g Zinkstaub, rührt 40 Min. im Eisbad, saugt dann unumgesetzten Zinkstaub ab (1,6 g) und wäscht gut mit 50%iger wäßriger Essigsäure nach. Das Filtrat wird im Vakuum eingedampft. Der Rückstand wird in Wasser vom pH-Wert 8 suspendiert, Schwefelwasserstoff durchgeleitet, abgesaugt und das Filtrat bei pH 10 mit Essigester extrahiert. Die Essigesterlösung wird mit Wasser vom pH 3 ausgerührt und diese wäßrige Lösung gefriergetrocknet.

Ausbeute 4,15 g
IR(Nujol); Carboxylbereich 1730; 1710, 1540 $cm^{-1}$.
Schmelzpunkt: ab etwa 130°C Zers.

Beispiel 7.3

*2-(2-tert.-Butoxycarbonylimino-4-thiazolin-4-yl)-2-aminoessigsäure:*

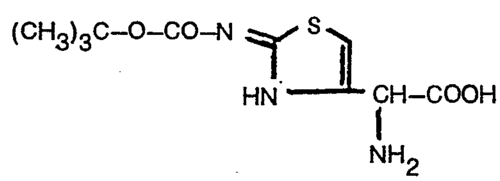

Die Lösung von 4,15 g einer nach Beispiel 7.2 dargestellten Substanz in 80 ml 50%igem wäßrigem Aethanol wird mit 2 n Natronlauge auf pH 1.3 gebracht und dieser pH solange gehalten, bis er sich nicht mehr ändert (etwa 15—20 Min.). Dann wird der pH mit Salzsäure auf 7,5 eingestellt und die Lösung im Vakuum eingedampft. Der Rückstand besteht aus der gesuchten Substanz, vermischt mit NaCl.

IR(Nujol; Carbonylbereich) 1715; 1600; 1545 $cm^{-1}$.

Beispiel 7.4

*2-(2-tert.-Butoxycarbonylimino-4-thiazolin-4-yl)-2-[(2-oxo-3-furylidenaminoimidazolidin-1-yl)-carbonylamino]-essigsäure:*

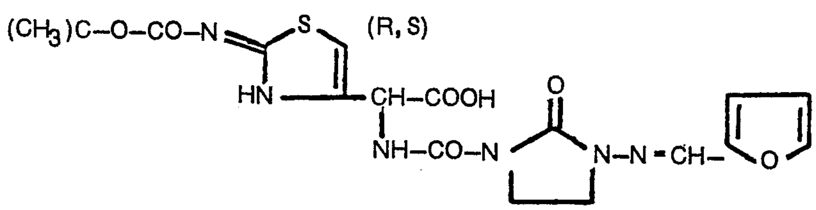

5,0 g einer nach Beispiel 7.3 hergestellten Substanz (enthält 2,0 g NaCl) werden in 70 ml 60%igem wäßrigem Tetrahydrofuran suspendiert bzw. gelöst und die Mischung mittels 2 n Natronlauge auf pH 7,5 gebracht. Dann werden 2,72 g 1-Chlorcarbonyl-2-oxo-3-furylidenaminoimidazolidin eingetragen und der pH mittels Natronlauge auf 7,5 gehalten. Wenn der pH sich nicht mehr ändert, wird das Tetrahydrofuran im Vakuum entfernt, mit 2 n Salzsäure bis pH 2,3 angesäuert, der ausgefallene Niederschlag abgesaugt und mit Wasser gewaschen. Der Niederschlag wird in heißem wäßrigem Aethanol suspendiert, abgekühlt und abgesaugt.

Ausbeute 3,8 g
Schmelzpunkt 226°C
NMR (60 MHz; in $d_6$-DMSO-$CD_3OD$)
7,55—7,8 (s breit; Furan 5-H; —N=CH—) 7,05 (s; Thiazolin 5-H) 6,75 (d; Furan 3-H) 6,5 (m; Furan 4-H) 5,4 (s; CH—N) 3,75 (s breit; —$CH_2$—$CH_2$) 1,45 (s; —$C(CH_3)_3$) ppm.

Beispiel 7.5

*7-{2-(2-tert.-Butoxycarbonylimino-4-thiazolin-4-yl)-2-[(2-oxo-3-furylidenaminoimidazolidin-1-yl)-carbonylamino]-acetamido}-cephalosporansäure:*

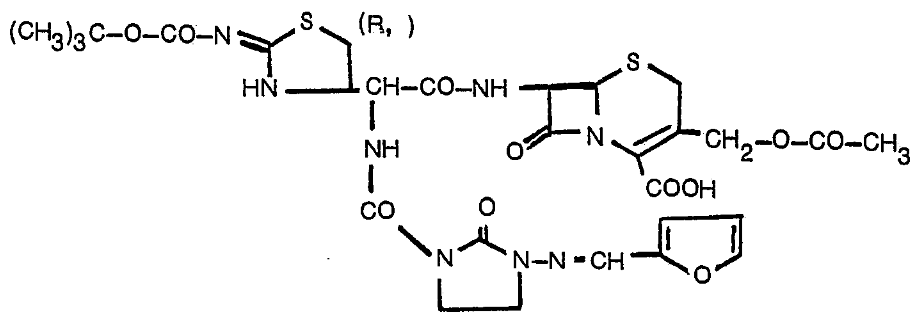

(Mischung A):

1,0 g der nach Beispiel 7.4 hergestellten Substanz wird in 30 ml Dichlormethan mit 0,29 ml Triäthylamin versetzt, die Lösung auf —40°C abgekühlt, mit 0,02 ml 3-Dimethylaminopropanol und 0,26 ml Chlorameisensäureäthylester versetzt, 60 Min. bei der gleichen Temperatur gerührt, weitere 0,13 ml Chlormeisensäureester und 0,15 ml Triäthylamin zugegeben und dieses nach 30 Min. wiederholt.

(Mischung B):

Zur im Eisbad gerührten Suspension von 0,77 g 7-Amino-cephalosporansäure in 20 ml Dichlormethan gibt man 2,0 ml Triäthylamin und 20 ml auf —10°C vorgekühltes Aceton. Die Mischung wird dann auf —20°C gekühlt.

Mischung A und B werden vereinigt und ohne Kältebad gerührt. Nach 2 Stunden wird mit Wasser verdünnt, derpH auf 7,5 gebracht, zweimal mit Essigester ausgeschüttelt und die wäßrige Phase auf pH 2 angesäuert. Es wird dreimal mit Essigester ausgeschüttelt, die vereinigten und getrockneten organischen Phasen im Vakuum eingedampft und der Rückstand im Exsiccator über $P_4O_{10}$ getrocknet.
Ausbeute 1,05 g

Beispiel 7.6

*7-{(2-Aminothiazol-4-yl)-2-[(2-oxo-3-furylidenaminoimidazolidin-1-yl)-carbonylamino]-acetamido}-cepholosporansäure:*

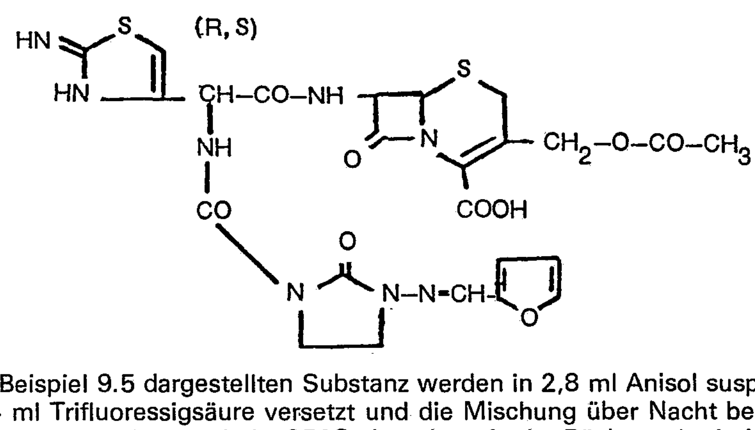

0,7 g der nach Beispiel 9.5 dargestellten Substanz werden in 2,8 ml Anisol suspendiert, in Eis/Wasser gekühlt, mit 14 ml Trifluoressigsäure versetzt und die Mischung über Nacht bei 4°C stehengelassen. Die Lösung wird dann im Vakuum bei —25°C eingedampft, der Rückstand mit Aether verrieben, abgesaugt und im Exsiccator über $P_4O_{10}$ getrocknet.
Ausbeute 0,6 g

IR-Spektrum(Carbonylbereich) 1740, 1715, 1660 und 1520—1500 $cm^{-1}$ (Nujol).

MHK—Werte

Verbindungen

| Keime | Beispiel 4 | Beispiel 5.5 | 1:1 Mischung Beispiel 4+ Beispiel 3.5 | 2:1 Mischung Beispiel 4+ Beispiel 5.5 |
|---|---|---|---|---|
| E.coli T 7 | 128—256 | 1 | 4 | 4—16 |
| E.coli A 261 | 32—64 | 1 | 4 | 4—16 |
| E.coli Neumann | 0,25 | 1 | ≤0,25 | ≤0,25 |
| E.coli 183/58 | 0,5—1 | 8—16 | 1 | 1 |
| E.coli F 14 | 256 | 8—16 | 16 | 64 |
| E.coli C 165 | ≤0,25 | 4 | 1 | 1 |
| E.coli 4 322 | 1 | 4 | 4 | 1 |
| Klebsiella 57 USA | 16 | 1 | 4 | 4—16 |
| Klebsiella 63 | 0,5—1 | 2—4 | 1 | 0,5—1 |
| Klebsiella 1852 | 128—256 | 4 | 16 | 4—16 |
| Klebsiella 6097 | 1 | 4 | 4 | 1 |
| Serratia 16001 | 0,5—1 | 4 | 2—4 | 1 |
| Serratia 16002 | 2—4 | 16 | 4 | 4—16 |
| Providencia 12012 | 1 | 4 | 16 | 1 |
| Prot.morg. 932 | 8—16 | 64 | 16 | 4—16 |
| Prot.morg. 11006 | 1 | 16 | 4 | 1 |
| Prot.vulg. 9023 | 1 | 8—16 | 4 | 1 |
| Prot.vulg. 1017 | 4 | 64 | 16 | 4—16 |
| Prot.rettg. 10007 | 1 | 16 | 4 | 1 |
| Staph.aur. 1756 | 128—256 | 256 | 128—256 | 256 |
| Staph.aur. 133 | 1 | 16 | 4 | 1 |
| Strept.faec. 27101 | 16 | 256 | 64 | 4—16 |
| Enteroc. ATCC 97900 | 32—64 | 256 | 128—256 | 64 |
| Psdm.aerug. F 41 | 16 | 256 | 64 | 4—16 |
| Psdm.aerug. Walter | 16 | 256 | 32 | 4—16 |

**Patentansprüche**

1. Verbindungen der Formel I

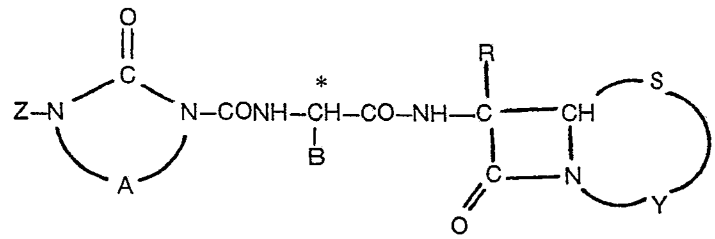

(I)

in welcher

Z 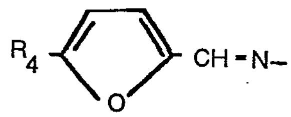 mit $R_4 = H, CH_3$, Cl und Br bedeutet und

R für Wasserstoff oder Niederalkoxy steht,
A für $—CH_2—CH_2—$, $—CH_2—CH_2—CH_2—$ oder 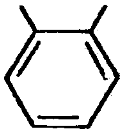 steht,

B für einen Furyl-, Thienyl-, 1,3,5-Trimethyl-pyrazolyl- oder 2-Aminothiazolylrest steht, und

Y für die Gruppen 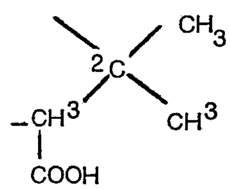 und 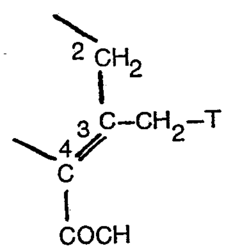 steht,

in welchen das Kohlenstoffatom, das die Gruppe —COOH trägt, an das Stickstoffatom des $\beta$-Lactamringes gebunden ist und
T für Wasserstoff, $—O—CH—CH_3$, Hydroxyl oder für gegebenenfalls durch Alkyl mit 1 bis 4 Kohlenstoffatomen oder $CF_3$ substituiertes Thiadiazolylthio oder Tetrazolylthio steht, wobei diese Verbindungen der Formel I bezüglich des Chiralitätszentrums $\overset{*}{C}$ in den beiden möglichen R- und S-Konfigurationen sowie als Gemische der daraus resultierenden Diastereomeren vorliegen können, und wobei die Verbindungen der Formel I bezüglich der Iminogruppe im Rest Z sowohl in der syn-Form als auch in der anti-Form vorliegen können und wobei diese Verbindungen der Formel I auch in den verschiedenen Hydratformen sowie in Form ihrer pharmazeutisch verwendbaren Salze vorliegen können.

2. Verbindungen der Formel I in der A für $—CH_2—CH_2—$ steht.

3. Verfahren zur Herstellung von Verbindungen der Formel I

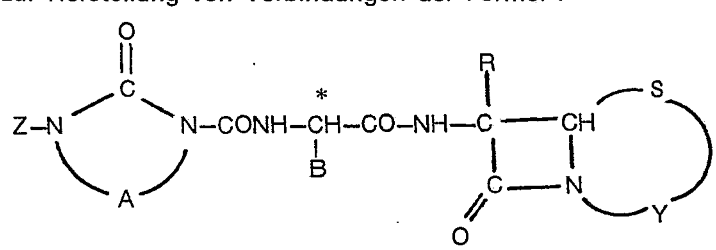

(I)

in der Z, A, $\overset{*}{C}$, B, R und Y oder deren Salze die in Anspruch 1 angegebene Bedeutung besitzen, dadurch gekennzeichnet, daß man Verbindungen der Formel II

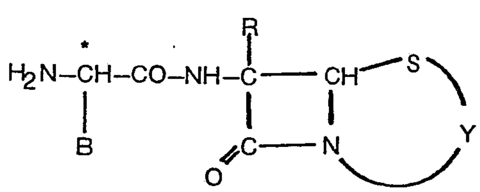

(II)

in welcher R, B, $\overset{*}{C}$ und Y die in Anspruch 1 angegebene Bedeutung haben, oder deren Salze mit Verbindungen der Formel III

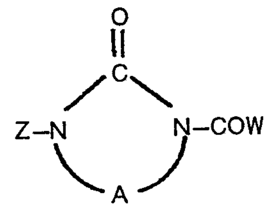

(III)

in welcher Z und A die in Anspruch 1 angegebene Bedeutung haben und

W für Halogen, Azid oder eine andere nukleofuge Abgangsgruppe steht,

in Gegenwart eines Lösungsmittels und gegebenenfalls eines Säurebindemittels bei Temperaturen von etwa −20 bis etwa +50°C umsetzt und die erhaltenen β-Lactam-Antibiotika gegebenenfalls in ihre Salze oder pharmazeutisch verwendbaren Ester überführt oder aus den erhaltenen Salzen gewünschtenfalls die freien Säuren herstellt.

4. Arzneimittel, gekennzeichnet durch einen Gehalt an einer Verbindung gemäß den Ansprüchen 1 bis 3.

**Revendications**

1. Composés de formule I:

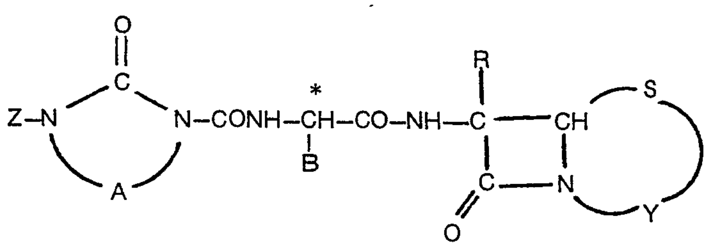

(I)

dans laquelle:

Z représente 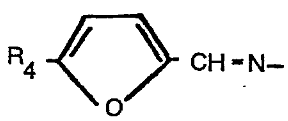 avec $R_4 = H, CH_3$, Cl et Br, et

R représente l'hydrogène ou un groupe alcoxy inférieur,

A représente $—CH_2—CH_2—$, $—CH_2—CH_2—CH_2—$ ou 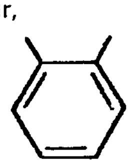

B représente un reste furyle, thiényle, 1,3,5-triméthyl-pyrazolyle ou 2-aminothiazolyle, et

Y représente les groupes: 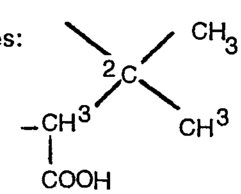 et 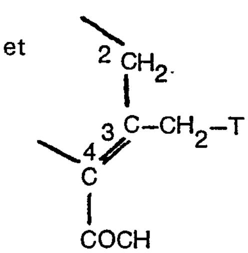

dans lesquels l'atome de carbone qui porte le groupe —COOH est relié à l'atome d'azote du cycle β-lactame, et

T représente l'hydrogène, un groupe $—O—CO—CH_3$, un groupe hydroxy ou un groupe thiadiazolylthio ou tétrazolylthio éventuellement substitué par des groupes alkyle en $C_1—C_4$ ou $CF_3$,

ces composés de formule I, relativement au centre de chiralite $\overset{*}{C}$, pouvant exister sous les deux configurations possibles R et S ou à l'état de mélanges des diastéréoisomères en résultant, et les composés de formule I, relativement au groupe imino du reste Z, pouvant exister sous la forme syn comme sous la forme anti, ces composés de formule I pouvant également exister sous les diverses formes d'hydrates et sous la forme de sels acceptables pour l'usage pharmaceutique.

2. Composés de formule I, dans laquelle A représente $—CH_2—CH_2—$.

3. Procédé de préparation des composés de formule I

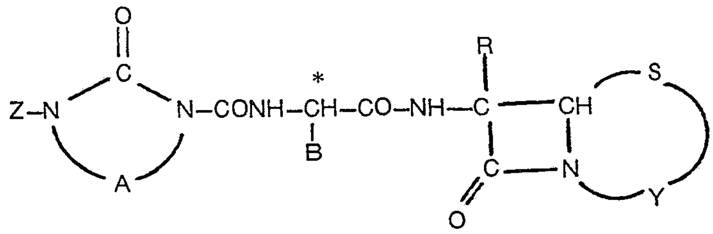

(I)

dans laquelle Z, A, $\overset{*}{C}$, B, R et Y ou leurs sels ont les significations indiquées ci-dessus, caractérisé en ce que l'on fait réagir des composés de formule II:

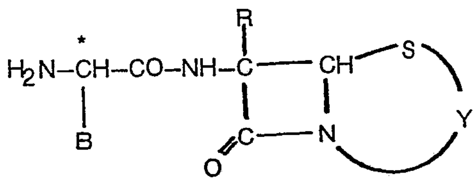

(II)

dans laquelle R, B, $\overset{*}{C}$ et Y ont les significations indiquées ci-dessus, ou leurs sels, avec des composés de formule III:

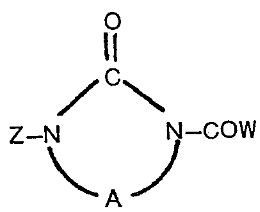

(III)

dans laquelle Z et A ont les significations indiquées ci-dessus et W représente un halogène, un groupe azide ou un autre groupe éliminable nucléofuge,
en présence d'un solvant et, le cas échéant, d'un agent fixant les acides à des températures allant de −20 à +50°C environ, et on convertit éventuellement les $\beta$-lactames antibiotiques obtenus en leurs sels ou en leurs esters acceptables pour l'usage pharmaceutique ou bien, à partir des sels obtenus, si on le désire, on prépare les acides libres.

4. Médicament, caractérisé en ce qu'il contient un composé selon les revendications 1 à 3.

**Claims**

1. Compounds of the formula I

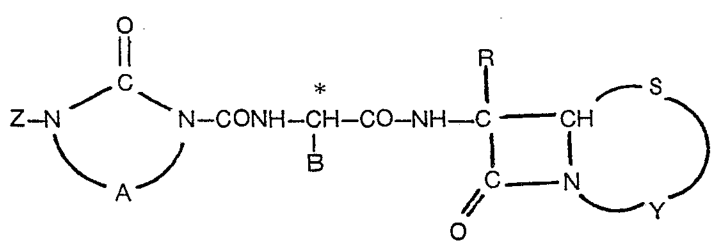

(I)

in which
Z denotes 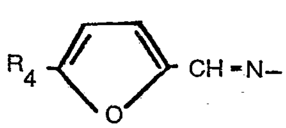 wherein $R_4 = H, CH_3, Cl$ and Br and

R represents hydrogen or lower alkoxy,
A represents $—CH_2—CH_2—$, $—CH_2CH_2—CH_2—$ or 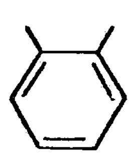

B represents a furyl, thienyl, 1,3,5-trimethyl pyrazolyl or 2-aminothiazolyl radical, and

Y represents the groups 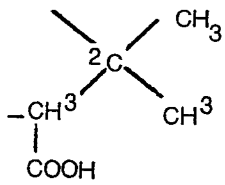 and 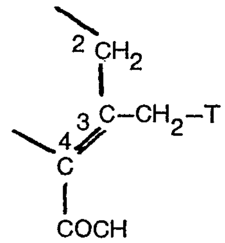

in which the carbon atom which carries the group —COOH is bonded to the nitrogen atom of the β-lactam ring and

T represents hydrogen, —O—CO—$CH_3$, hydroxyl or thiadiazolylthio or tetrazolylthio which is optionally substituted by alkyl with 1 to 4 carbon atoms or $CF_3$, it being possible for these compounds of the formula I to exist in the two possible R and S configurations, with respect to the chirality centre $\overset{*}{C}$, and as mixtures of the diastereomers resulting therefrom, and it being possible for the compounds of the formula I to exist both in the syn-form and in the anti-form, with respect to the imino group in the radical Z, and it also being possible for these compounds of the formula I to exist in the various hydrate forms as well as in the form of their pharmaceutically usable salts.

2. Compounds of the formula I in which A represents —$CH_2$—$CH_2$—.

3. Process for the preparation of compounds of the formula I

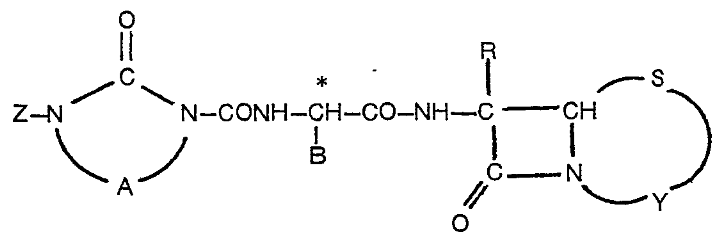

(I)

in which

Z, A, $\overset{*}{C}$, B, R and Y, or salts thereof, have the meaning indicated above, characterised in that compounds of the formula II

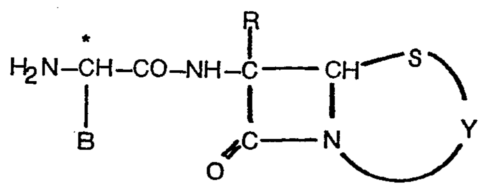

(II)

in which

R, B, $\overset{*}{C}$ and Y have the meaning indicated above, or salts thereof, are reacted with compounds of the formula III

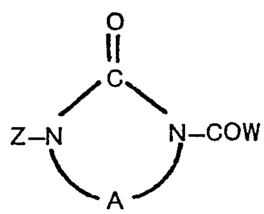

(III)

in which

Z and A have the meaning indicated above and

W represents halogen, azide or another nucleofugic leaving group,

in the presence of a solvent and optionally of an acid-binding agent at temperatures of about −20 to about +50°C and the resulting β-lactam antibiotics are optionally converted into their salts or pharmaceutically usable esters, or, if desired, the free acids are prepared from the resulting salts.

4. Medicaments, characterised in that they contain a compound according to Claims 1 to 3.